**Europäisches Patentamt**

**European Patent Office**

**Office européen des brevets**

(19)

(11) Veröffentlichungsnummer: **0 218 785 B1**

# EUROPÄISCHE PATENTSCHRIFT

(12)

(45) Veröffentlichungstag der Patentschrift:
23.01.91 Patentblatt 91/04

(51) Int. Cl.⁵: **A61M 1/00, A61M 1/02**

(21) Anmeldenummer: **86107663.6**

(22) Anmeldetag: **17.05.83**

(54) **Gerät zur Aufnahme und Reinfusion von Blut.**

(30) Priorität: **17.05.82 DE 3218561**

(43) Veröffentlichungstag der Anmeldung:
**22.04.87 Patentblatt 87/17**

(45) Bekanntmachung des Hinweises auf die
Patenterteilung:
**23.01.91 Patentblatt 91/04**

(84) Benannte Vertragsstaaten:
**AT BE CH FR GB IT LI NL SE**

(56) Entgegenhaltungen:
DE-B- 1 766 217
FR-A- 1 108 782
FR-A- 2 195 457
US-A- 3 774 611
US-A- 4 033 345
US-A- 4 335 717

(61) Veröffentlichungsnummer der früheren
Anmeldung nach Art. 76 EPü: **0094682**

(73) Patentinhaber: **SOLCO BASEL AG**
**Rührbergstrasse 21**
**CH-4127 Birsfelden (CH)**

(72) Erfinder: **Marx, Günter H., Dr.-Ing.**
**G.-Caracciola-Strasse 10**
**D-8035 Gauting (DE)**

(74) Vertreter: **LOUIS, PÖHLAU, LOHRENTZ &**
**SEGETH**
**Ferdinand-Maria-Strasse 6**
**D-8130 Starnberg (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Gerät zur Aufnahme und Reinfusion von eigenem Blut eines Patienten, z.B. während einer Operation, mit den Merkmalen gemäß dem Oberbegriff des Patentanspruches 1.

Bei Operationen, insbesondere Herzoperationen, aber auch bei schweren Verletzungen kommt es sehr häufig zu Blutverlusten des Patienten, die durch eine sofortige Transfusion kompensiert werden müssen, wenn das Leben des Patienten nicht gefährdet werden soll. Insbesondere bei Operationen erfolgt der Blutverlust in einer Weise, daß das verlorene Blut noch zur Verfügung steht, nämlich in Form von Blutansammlungen in Körperhöhlen des Patienten, die für den Chirurgen zugänglich sind. Es ist deshalb schon bekannt, das Blut aus derartigen Blutansammlungen wieder aufzunehmen und demselben Patienten zu reinfundieren. Ein solcher Vorgang des Aufsammelns und der erneuten Reinfusion von bzw. zu ein und demselben Patienten wird auch als intraoperative Autotransfusion bezeichnet.

Zur Durchführung der Autotransfusion ist bereits ein Gerät bekannt (US-A 40 47 526), bei dem das Blut des Patienten zunächst in einen formsteifen Behälter durch einen mittels einer Unterdruckquelle darin erzeugten Unterdruck eingesaugt und anschließend aus diesem formsteifen Behälter mittels einer Blutpumpe dem Patienten reinfundiert wird. In einer besonderen Ausführungsform dieses bekannten Geräts ist die an den formsteifen Behälter angeschlossene Blutpumpe durch einen axial zusammendrückbaren Faltenbalg od.dgl. gebildet, der lösbar mit dem formsteifen Behälter verbunden werden kann. Um das in dem formsteifen Behälter befindliche Blut in den Faltenbalg einsaugen zu können, wird dieser zunächst auf sein Minimalvolumen zusammengedrückt und an einem Auslaß des formsteifen Behälters mittels eines Schlauchstückes od.dgl. befestigt, wobei mittels einer Schlauchklemme der zusammengedrückte Zustand des Faltenbalges vorläufig aufrecht erhalten werden kann. Wird die Schlauchklemme abgezogen, so dehnt sich der Faltenbalg aufgrund der ihm innewohnenden Elastizität aus und überwindet dadurch den in dem formsteifen Behälter herrschenden Unterdruck, so daß Blut in den Faltenbalg eingesaugt werden kann. Nach seiner Füllung wird der Faltenbalg von dem formsteifen Behälter gelöst, umgedreht, so daß sein Anschluß unten liegt, und an einer hierfür an seinem Boden vorgesehenen Lasche aufgehängt. In diesem Zustand wird auf dem Weg einer herkömmlichen Transfusion die Reinfusion vorgenommen. Ein wesentlicher Nachteil dieses bekannten Geräts besteht darin, daß die Reinfusion des in dem Faltenbalg aufgenommenen Blutes nur auf dem Weg einer herkömmlichen Transfusion vorgenommen wird. Da eine Autotransfusion der hier besprochenen Art jedoch immer dann vorgenommen wird, wenn grössere Blutverluste des Patienten vorliegen, ist es zugleich von wesentlicher Bedeutung, daß dem Patienten das Blut auch in kürzester Zeit wieder reinfundiert wird, da andernfalls während eines erheblichen Zeitraumes ein beträchtliches und daher lebensgefährdendes Defizit an Körperblut entstehen kann. Dies ist nur mit einer Druckinfusion des aufgenommenen Blutes zu erreichen, für die aber das bekannte Gerät nicht vorgesehen ist und welche überdies damit ohne die Gefahr einer Luftembolie des Patienten nicht ausführbar ist. Ein weiterer Nachteil besteht darin, daß das Blut mit einer verhältnismässig grossen blutfremden Oberfläche in Kontakt kommt, nämlich mit der Oberfläche des formsteifen Behälters und mit derjenigen des Faltenbalges. Das kann ebenfalls zu einer Beeinträchtigung des Blutes führen. Schließlich ist auch von Nachteil, daß beim Ansaugen des Blutes aus dem formsteifen Behälter in den Faltenbalg in letzterem ein Unterdruck herrschen muß, der noch geringer ist als derjenige in dem formsteifen Behälter, um ein Ansaugen des Blutes überhaupt zu ermöglichen. Durch diesen kräftigen Unterdruck werden jedoch die roten Blutkörperchen belastet und können geschädigt werden. Darüber hinaus ist zu seiner Erzeugung eine starke Federspannung des Faltenbalges erforderlich, die sich beim Zusammenpressen des Faltenbalges vor dessen Verbindung mit dem formsteifen Behälter als hinderlich erweist.

Es ist weiterhin auch schon ein Gerät zur Aufnahme und Reinfusion von eigenem Blut eines Patienten gemäß dem Oberbegriff des Patentanspruches 1 bekannt, das eine insgesamt aus drei Behältern bestehende Behälteranordnung umfasst (US-A 40 33 345). Hierbei handelt es sich um einen starren Behälter, der durch eine Saugpumpe evakuierbar ist, um einen nachgeschalteten verformbaren Behälter, der als Blutpumpe wirksam ist, und um einen diesen verformbaren Behälter umgebenden starren Behälter, der alternierend mit Überdruck oder Unterdruck beaufschlagbar ist, um den verformbaren Behälter als Pumpe wirken zu lassen. In einer Trennwand zwischen dem erstgenannten starren Behälter und dem verformbaren Behälter ist ein in Einströmöffnung zu letzterem öffnendes Rückschlagventil angeordnet; ein in Ausströmrichtung öffnendes Rückschlagventil ist in der Auslaßleitung aus dem verformbaren Behälter angeordnet. Zwar ist es mit diesem bekannten Berät im Unterschied zu dem vorstehend besprochenen Gerät nach der US-A 40 47 526 bereits möglich, eine Druckinfusion des Blutes vorzunehmen, jedoch hat es im übrigen die gleichen Nachteile wie dieses. Diese bestehen in der verhältnismässig grossen blutfremden Oberfläche, mit der das Blut in Kontakt kommt, mit dem kräftigen Unterdruck, der zum Einsaugen des Blutes aus dem vorgeschalteten starren Behälter über das in Einlaßrichtung öffnende Rückschlagventil in den verformbaren Behälter notwendig ist, sowie in der durch die Doppelanordnung der starren Behälter notwendigerweise grossen Abmessung, die die Handhabung erschwert.

Es ist weiterhin auch schon eine Saug-Dränage-Vorrichtung in Gestalt eines Faltenbalges bekannt, durch die Sekrete aus der Umgebung einer Wunde eines Patienten abgesaugt werden können (DE-B 17 66 217). Dieser Faltenbalg wird ebenfalls vor der Benutzung auf sein minimales Volumen zusammengedrückt und kann sich unter Erzeugung eines Unterdruckes in seinem Inneren aufgrund der ihm innewohnenden Eigenelastizität sowie aufgrund der Elastizität eines ihn aufweitenden Spannbügels ausdehnen. Um evtl. eingedrungene Luft zu beseitigen, weist der Faltenbalg eine Luftauslaßvorrichtung auf, durch deren Öffnen der Faltenbalg wieder zusammengedrückt und überschüssige Luft abgelassen werden kann. Diese bekannte Vorrichtung ist jedoch für eine Bluttransfusion weder bestimmt noch geeignet.

Der Erfindung liegt die Aufgabe zugrunde, ein Gerät zur Durchführung der Autotransfusion zu schaffen, durch welches das Blut auf kurzem Wege und daher ohne eine Schädigung vom Patienten aufgenommen werden und dem Patienten auf ebenso kurzem Weg und ohne Schädigung in kurzer Zeit wieder zugeführt werden kann.

Die Lösung dieser Aufgabe ist im Kennzeichen des Patentanspruches 1 angegeben.

Bei dem Gerät nach der Erfindung wird der Ansaugeffekt durch die aufgrund der vorhandenen Eigenelastizität und/oder Federkraft bewirkte Expansion des Behälters, beispielsweise eines Faltenbalges, von einem ursprünglich zusammengedrückten Zustand aus erzeugt, wobei zur Blutschonung der Sog durch das Ausmaß der Zusammendrückung des Behälters steuerbar ist. Der Werkstoff und die Wanddicke des Behälters, z.B. des Faltenbalges, sind so gewählt, daß der bei der Expansion auftretende Unterdruck im Inneren nicht zu einem Einstülpen der seitlichen Wände des Behälters führt, was aufgrund der inherenten höheren Steifigkeit der Faltenstruktur bei einem Faltenbalg unschwierig erreichbar ist. Die in den Anschlüssen vorgesehenen Rückschlagventile sorgen dafür, daß in den Behälter eingesaugtes Blut nicht wieder zurückströmen kann und daß der zum Ansaugen erforderliche Unterdruck nicht durch einströmende Luft von dem später der Reinfusion dienenden Anschluß her gebrochen wird. Die Reinfusion erfolgt durch manuelles oder mechanisches Zusammendrücken des Behälters in axialer Richtung. Dieses Zusammendrücken kann auf verschiedenartige Weise erfolgen, z.B. manuell durch den Anästhesisten, oder durch Einsetzen in eine Druckvorrichtung, z.B. ein aufblasbare Druckmanschette od.dgl. Insbesondere das manuelle Zusammendrücken des Behälters hat den Vorteil, daß der Anästhesist den Druck gefühlvoller steuern kann als dies durch die Zuführung von Druckluft möglich ist.

Die beschriebene Ventilanordnung in dem Behälter ermöglicht es dem Chirurgen, bei nur teilweise gefülltem Behälter (nach Ansaugen von Blutschaum oder Luft) diesen von Hand erneut zusammenzudrücken, so daß die darin enthaltene Luft durch das nach außen hin öffnende Rückschlagventil ausgedrückt wird und durch erneutes Loslassen des Behälters der nunmehr von Luft befreite Innenraum ebenfalls noch mit Blut gefüllt werden kann. Um in einem solchen Fall zu verhindern, daß beim Hinausdrücken der enthaltenen Luftmenge auch zugleich Blut unerwünschterweise ausgepresst wird, ist nach einer vorteilhaften Weiterbildung vorgesehen, daß dem dem weiteren Anschluß zugeordneten Rückschlagventil ein in Ausströmrichtung aus dem Behälter sperrendes Flüssigkeits-Rückschlagventil vorgeschaltet ist, dessen Sperrfunktion nur bei einer Flüssigkeit eintritt. Ein solches Flüssigkeits-Rückschlagventil ist z.B. ein vor dem Anschluß im Inneren des Behälters angeordnetes verschwenkbares Folienkissen oder eine Kugel, die schwerer als Luft, jedoch leichter als eine Flüssigkeit ist.

Steigt somit das Blut bis zu der Öffnung des weiteren Anschlusses hin durch Zusammendrücken des Behälters an, so schwimmt das Folienkissen bzw. die Kugel, die zuvor beim Ausdrücken der Luft keine Sperrwirkung entfalten können, auf und bewirkt ein Verschließen des weiteren Anschlusses. Auch ein hydrophobes Filter ist als Ventil anwendbar.

Bei dem erfindungsgemässen Gerät wird zum Ansaugen und zur anschließenden Reinfusion des Blutes ein und derselbe Behälter verwendet. Folglich ist die mit dem Blut in Kontakt kommende Behälteroberfläche kleiner als bei dem eingangs geschilderten bekannten Gerät. Darüber hinaus herrscht in dem Behälter nur derjenige Druck, der zum Ansaugen des Blutes unmittelbar vom Patienten her erforderlich ist und folglich keinen so hohen Wert annehmen muß, wie dies bei dem bekannten Gerät zur Überwindung des Unterdruckes in dem formsteifen Behälter erforderlich ist. Das Blut wird daher in dem erfindungsgemässen Gerät schonender behandelt.

Es versteht sich, daß der Behälter aus physiologisch unbedenklichen Werkstoffen, z.B. Polyäthylen, Polyurethan oder Polyamid, gefertigt ist und vorzugsweise auch durchscheinend oder durchsichtig ist, damit die Standhöhe des Blutes darin überwacht werden kann.

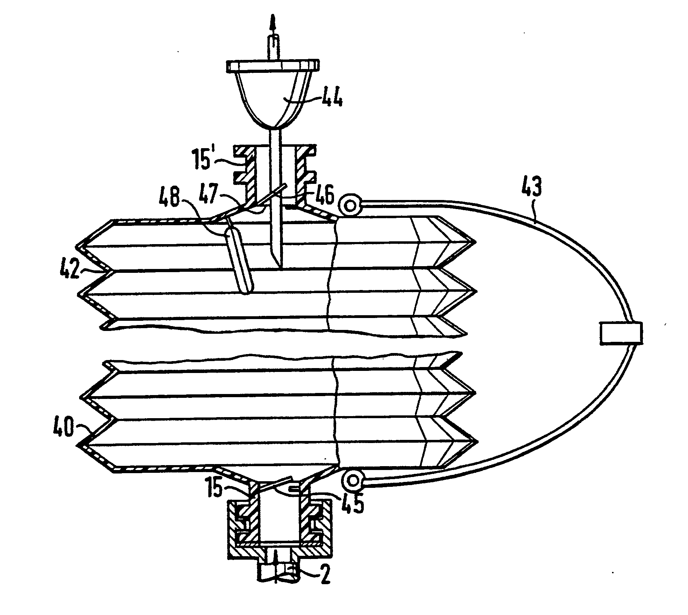

Weitere Vorteile und Merkmale der vorliegenden Erfindung ergeben sich aus der nachfolgenden Beschreibung eines bevorzugten Ausführungsbeispieles anhand der beiliegenden Zeichnung. Bezüglich Einzelheiten, die in der nachfolgenden Beschreibung nicht ausdrücklich erwähnt, jedoch aus der Zeichnung ersichtlich sind, wird auf die Zeichnung Bezug genommen.

Die Zeichnung zeigt einen Längsschnitt durch die Behälteranordnung des erfindungsgemässen Geräts. Als Behälter zur Aufnahme des zu reinfundierenden Blutes ist ein Faltenbalg 40 vorgesehen, dessen Wandstärke und Werkstoff im Bereich der Falten so gewählt sind, daß diese auch bei einem im Inneren des Faltenbalges 40 herrschenden Unterdruck nicht in radialer Richtung zusammengedrückt werden. Aufgrund einer entsprechenden Wandstärke auch im Bereich der Kanten 42, an denen die Falten sich schneiden, weist er eine erhöhte Eigenelastizität auf. Diese Eigenelastizität wirkt in einem Sinne, daß nach einem Zusammendrücken des Faltenbalges 40 dieser sich selbsttätig wieder in den expandierten Zustand zurückbewegt. Diese Eigenelastizität kann durch eine an den Stirnflächen des Faltenbalges eingehängte Spreizfeder 43, die den Faltenbalg 40 aufzuziehen trachtet, unterstützt werden.

Der Faltenbalg 40 weist Anschlüsse 15,15' auf, von denen einer mit einer Saugleitung 2 koppelbar ist, um Blut in das Innere des Faltenbalges 40 einsaugen zu können. Der andere Anschluß 15' dient ausschließlich bei der Reinfusion des Blutes dem Anschluß eines Druckinfusionsbestecks, von dem das Anschlußrohr und der zugehörige Dom eines Blutfilters 44 angedeutet sind.

In dem zur Saugleitung 2 gerichteten Anschluß 15 ist ein Rückschlagventil 45 angeordnet, das in Richtung zum Innenraum des Faltenbalges 40 öffnet, in der Gegenrichtung jedoch sperrt. In dem genenüberliegenden Anschluß 15', der später mit dem Druckinfusionsbesteck verbunden wird, ist ebenfalls ein Rückschlagventil 46 angeordnet, das in einer Strömungsrichtung aus dem Innenraum des Faltenbalges 40 heraus öffnet, in der Gegenrichtung jedoch sperrt. Zusätzlich ist in der Nähe der zu diesem Anschluß 15' führenden Öffnung 47 ein leichtes luftgefülltes Folienkissen 48 derart befestigt, daß es unter seinem Eigengewicht in der aus der Zeichnung ersichtlichen Weise nach unten hängt und auch durch eine Luftströmung aus dem Inneren des Faltenbalges 40 durch das Rückschlagventil 46 hindurch nicht vor die Öffnung 47 bewegt werden kann.

Die Wirkungsweise des Geräts ist folgende : Zunächst wird der Faltenbalg 40 durch manuelle Einwirkung axial auf sein geringstmögliches Volumen zusammengepresst, wozu sowohl die Eigenelastizität der Faltenbalgwandung als auch diejenige der ggf. vorhandenen Spreizfeder 43 überwunden werden muß. In diesem Zustand wird die Saugleitung 2 abgeklemmt, so daß in das Innere des Faltenbalges 40 keine Luft eintreten kann und deshalb der zusammengedrückte Zustand des Faltenbalges 40 von selbst erhalten bleibt. Wird nunmehr der nicht gezeigte Saugkopf der Saugleitung 2 in eine Blutansammlung getaucht und die die Saugleitung 2 verschließende Klemme langsam freigegeben, so dehnt sich der Faltenbalg aufgrund seiner Eigenelastizität sowie aufgrund der Vorspannung durch die ggf. vorhandene Spreizfeder 43 in axialer Richtung aus. Dadurch vergrössert sich sein Volumen gegenüber dem ursprünglichen zusammengedrückten Zustand und Blut wird durch die Saugleitung 2 sowie durch das in dieser Richtung öffnende Rückschlagventil 45 eingesaugt. Durch das Rückschlagventil 46 wird während dieser Phase der Zutritt von Luft unterbunden, so daß die Unterdruckwirkung nicht gebrochen wird. Ist durch Unachtsamkeit oder durch ein gewisses Lecken (Ansaugen von Blutschaum) eine bestimmte Luftmenge in das Innere des Faltenbalges 40 mit eingedrungen, so daß dieser nicht vollständig mit Blut gefüllt ist, obwohl er bereits maximal expandiert ist, so kann der Chirurg durch erneutes manuelles Zusammendrücken des Faltenbalges 40 diese Luftmenge über den Anschluß 15' mit dem Rückschlagventil 46 entfernen. Dabei verhindert das Folienkissen 48, daß zusätzlich Blut durch das Rückschlagventil 46 unerwünschterweise hindurchgelangt. Denn das Folienkissen schwimmt auf dem Blutspiegel auf und wird dadurch bei dessen Ansteigen vor die Öffnung 47 verschwenkt, so daß es diese dadurch abdichtet.

Ist der Faltenbalg 40 in der geschilderten Weise mit Blut gefült, so wird mit dem für die Reinfusion bestimmten Anschluß 15' das Druckinfusionsbesteck, ggf. mit einem vorgeschalteten Blutfilter, verbunden. Die Nadel des Druckinfusionsbestecks ist zuvor bereits in ein Blutgefäß des Patienten eingestochen und befestigt worden. Die Reinfusion des im Faltenbalg 40 enthaltenen Blutes zum Patienten erfolgt nun durch manuelles oder mechanisches Zusammendrücken des Faltenbalges 40 in axialer Richtung. Von Bedeutung ist dabei, daß durch das Anschlußrohr des Blutfilters bzw. durch das entsprechende Rohr des Druckinfusionsbestecks das Folienkissen 48 zurückgestossen wird, um dessen Sperrwirkung zu beseitigen.

Die Anschlüsse 15,15' können abweichend von dem gezeigten Ausführungsbeispiel auf der gleichen Stirnseite angeordnet sein, wobei dann allerdings durch eine entsprechende Verlängerung ins Innere Kurzschlüsse vermieden werden müssen. Außerdem ist es möglich, die Funktion der Rückschlagventile 45, 46 an eine andere Stelle zu legen, z.B. in die daran angeschlossenen Leitungen oder in kurze Leitungsstücke, die dann selbst die Anschlüsse 15,15' bilden. Auch ist die Erfindung keineswegs auf die in dem Ausführungsbeispiel gezeigte Art von Anschlußstutzen beschränkt, und die Eigenelastizität des Faltenbalges 40 kann auch durch unmittelbar in das Kunststoffmaterial des Faltenbalges eingebettete Metallteile oder Federn bewirkt sein.

Es ist auch denkbar, den Faltenbalg 40 mit einem Behälter derart einstückig zu kombinieren, daß der untere Teil reine Behälterfunktion hat und nur der obere Teil als Faltenbalg ausgebildet ist. Für diese Ausführung ist es auch von Vorteil, den unteren Anschluß 15 nach innen zu, z.B. mittels eines Rohrstückes, so weit zu verlängern, daß angesaugtes Blut/Luft-Gemisch nicht durch das bereits vorhandene Blutreservoir hindurchtritt.

Der eigentliche Zweck des hier beschriebenen Gerätes liegt in der Autotransfusion, d.h. bei der Aufnahme und Reinfusion von eigenem Blut eines Patienten, das aus Körperhöhlen od.dgl. angesaugt wird. Jedoch ist das Gerät aufgrund seiner Konstruktion nicht ausschließlich für die Autotransfusion verwendbar, sondern kann auch für eine Wunddränage und sonstige Dränagen und insbesondere auch für die Blutspende verwendet werden.

## Ansprüche

1. Gerät zur Aufnahme und Reinfusion von eigenem Blut eines Patienten, z.B. während einer Operation, mit einer Behälteranordnung (40), die einen Sauganschluß (15) mit einer daran anschließbaren Saugleitung (2) zum Absaugen von Blut in die Behälteranordnung sowie einen weiteren Anschluß (15′) zur Reinfusion des in der Behälteranordnung befindlichen Blutes aufweist, und mit einem Paar von Rückschlagventilen (45, 46), von denen das dem Sauganschluß (15) zugeordnete in Einströmrichtung und das dem weiteren Anschluß (15′) zugeordnete in Ausströmrichtung aus der Behälteranordnung öffnet, dadurch gekennzeichnet, daß die Behälteranordnung durch einen einzigen Behälter (40) gebildet ist, der in einer Achsrichtung zusammendrückbar, senkrecht zu dieser Achsrichtung jedoch relativ formsteif ist und durch die Eigenelastizität seines Werkstoffes und/oder durch eine an seinen Stirnseiten angreifende Federkraft (43) aus dem zusammengedrückten in einen auseinandergezogenen Zustand rückführbar ist, und daß der Sauganschluß (15) unmittelbar an dem zusammendrückbaren Behälter angebracht ist.

2. Gerät nach Anspruch 1, dadurch gekennzeichnet, daß dem dem weiteren Anschluß (15′) zugeordneten Rückschlagventil (46) ein in Ausströmrichtung aus dem Behälter (40) sperrendes Flüssigkeits-Rückschlagventil (48) vorgeschaltet ist, dessen Sperrfunktion nur bei einer Flüssigkeit eintritt.

3. Gerät nach Anspruch 2, dadurch gekennzeichnet, daß das Flüssigkeits-Rückschlagventil (48) ein im Inneren des Behälters (40) vor der zu dem weiteren Anschluß (15′) führenden Öffnung (47) angeordnetes verschwenkbares Folienkissen, eine Kugel oder ein hydrophobes Filter ist.

## Claims

1. Apparatus for collection and reinfusion of a patient's own blood, for example during an operation, comprising a container means (40) wich has a suction connection (15) with a suction line (2) connectable thereto, for sucking blood into the container means, and a further connection (15′) for reinfusion of the blood in the container means, and further comprising a pair of check valves (45, 46) of which that which is associated with the suction connection (15) opens in the direction of flow into the container means and that associated with the further connection (15′) opens in the direction of flow out of the container means, characterised in that the container means is formed by a single container (40) which is compressible in an axial direction but relatively rigid perpendicularly to said axial direction and can be returned from the compressed condition into an expanded condition by the natural resiliency of its material and/or by a spring force (43) engaging the container at the ends thereof, and that the suction connection (15) is disposed directly on the compressible container.

2. Apparatus according to claim 1 characterised in that disposed upstream of the check valve (46) associated with the further connection (15′) is a liquid check valve (48) which closes in the direction of flow out of the container (40) and the closure function of which occurs only in relation to a liquid.

3. Apparatus according to claim 2 characterised in that the liquid check valve (48) is a pivotable foil cushion, a ball or a hydrophobic filter, disposed in the interior of the container (40) upstream of the opening (47) leading to the further connection (15′).

## Revendications

1. Appareil pour la récolte et la réinjection du propre sang d'un patient, par exemple pendant une opération, comprenant un agencement de réservoir (40) qui présente un raccord d'aspiration (15) équipé d'une conduite d'aspiration (2) qui peut y être raccordée pour l'aspiration de sang dans l'agencement de réservoir, ainsi qu'un autre raccord (15′) pour la réinjection du sang situé dans l'agencement de réservoir, et comprenant une paire de clapets antiretour (45,46) dont celui qui est adjoint au raccord d'aspiration (15) s'ouvre dans le sens d'écoulement vers l'intérieur et dont celui qui est adjoint à l'autre raccord (15′) s'ouvre dans le sens d'écoulement vers l'extérieur de l'agencement de réservoir, caractérisé en ce que l'agencement de réservoir est formé par un réservoir unique (40) qui est compressible dans une direction axiale, qui est cependant de forme relativement rigide perpendiculairement à cette direction axiale et qui peut être ramené de l'état comprimé à un état étiré par l'élasticité propre de son matériau et/ou par une force de ressort (43) agissant sur ses côtés frontaux, et en ce que le raccord d'aspiration (15) est appliqué directement au réservoir compressible.

2. Appareil suivant la revendication 1, caractérisé en ce qu'en amont du clapet antiretour (46) adjoint à l'autre raccord (15′) est monté un clapet antiretour pour liquide (48) qui produit un blocage dans le sens de sortie du réservoir (40) et dont la fonction de blocage ne se produit que pour un liquide.

3. Appareil suivant la revendication 2, caractérisé en ce que le clapet antiretour pour liquide (48) est un coussin de feuilles qui peut pivoter, une bille ou un filtre hydrophobe, agencé à l'intérieur du réservoir (40) devant l'orifice (47) conduisant à l'autre raccord (15').